# EUROPÄISCHE PATENTANMELDUNG

(11) **EP 4 664 595 A1**
(43) Veröffentlichungstag der Anmeldung: **17.12.2025**
(21) Anmeldenummer: 25182371.2
(22) Anmeldetag: 12.06.2025
(51) Int. Cl.: H01M 10/0569, C07D 317/12, C07D 493/04

(54) **BATTERIEELEKTROLYTE MIT LÖSUNGSMITTELN UMFASSEND CYCLISCHE ACETALE**

(30) Priorität: 13.06.2024 DE 102024116584
(71) Anmelder: Forschungszentrum Jülich GmbH, 52425 Jülich (DE)
(72) Erfinder: Krämer, Susanna, 47229 Duisburg (DE); Grünebaum, Mariano, 59387 Ascheberg (DE); Winter, Martin, 48149 Münster (DE)
(74) Vertreter: Michalski Hüttermann & Partner Patentanwälte mbB

(57) **Zusammenfassung**

Die vorliegende Erfindung betrifft einen lösungsmittelbasierten Elektrolyt für einen elektrochemischen Energiespeicher, aufweisend wenigstens die folgenden Bestandteile:
- wenigstens ein Lösungsmittel; und
- wenigstens ein Leitsalz, wobei

wenigstens ein Lösungsmittel der folgenden Formel (1) entspricht: 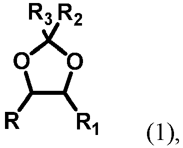 wobei
- R und R₁ gleich oder verschieden sind und ausgewählt sind aus der Gruppe bestehend aus H, CH₃, der Struktur gemäß Formel (3) und der Struktur gemäß Formel (4) oder R und R₁ gemeinsam die Struktur gemäß Formel (2) ausbilden, wobei
- die Formel (2) der folgenden Struktur entspricht: 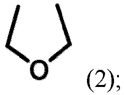 - die Formel (3) der folgenden Struktur entspricht: 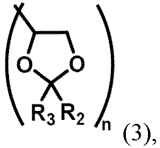 wobei n eine ganze Zahl von 1 bis 10 ist;
- die Formel (4) der folgenden Struktur entspricht:

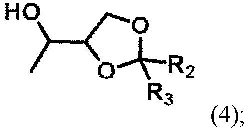

wobei in den Formeln (1), (3), und (4) R₂ und R₃ gleich oder verschieden sind und ausgewählt sind aus der Gruppe bestehend aus H, (CH₂)ₓ-CH₃, wobei x eine ganze Zahl von 0 bis 9 ist.

## Beschreibung

Die vorliegende Erfindung betrifft lösungsmittelbasierte Elektrolyte für elektrochemische Energiespeicher, insbesondere für Lithium-Ionen-Batterien aufweisend cyclische Acetale. Die vorliegende Erfindung betrifft ferner elektrochemische Energiespeicher aufweisend derartige Elektrolyte als auch die Verwendung von cyclischen Acetalen als Lösungsmittel in elektrochemischen Energiespeichern.

Elektrochemische Energiespeicher, wie etwa Lithium-Ionen-Batterien, werden für eine Vielzahl an mobilen und stationären Anwendungen benötigt. Bestandteil derartiger Energiespeicher ist dabei ein Elektrolyt, wobei lösungsmittelbasierte Elektrolyte als auch Feststoffelektrolyte bekannt sind. Dabei sollte der Elektrolyt sowohl ausreichend stabil sein, um auch Anwendungen in Hochvoltanwendungen standzuhalten, als auch sollten Elektrolyte geeignete ionische Eigenschaften aufweisen, wie etwa eine ausreichende Leitfähigkeit beziehungsweise Teilleitfähigkeit.

Hinsichtlich der Elektrolyte sind im Bereich der wässrigen Elektrolyte insbesondere hochkonzentrierte, sogenannte Wasser-in-Salz Elektrolyte bekannt. Dabei werden jedoch hohe Konzentrationen eines Leitsalzes, etwa in einem Bereich von 21mol/kg LiTFSI in Wasser verwendet, um ein ausreichendes elektrochemisches Stabilitätsfenster zu erzielen. Weiterhin sind etwa in Lithium-Ionen-Batterien nicht-wässrige, organische Elektrolyte mit einem Leitsalz, wie etwa LiPF₆ und carbonat-basierten Lösungsmitteln bekannt. Derartige Lösungsmittel können etwa eine cyclische oder lineare Struktur aufweisen und etwa Dimethylcarbonat, Ethylmethylcarbonat oder auch Ethylencarbonat umfassen. Weiterhin als Lösungsmittel in Elektrolyten bekannt sind Ether, Nitrile oder wie bereits erwähnt Wasser.

Der Stand der Technik bei wässrigen Elektrolyten bietet jedoch noch Verbesserungspotential insbesondere hinsichtlich ionischer Eigenschaften und elektrochemischer Stabilität bei gleichzeitig reduziertem Einsatz eines Leitsalzes.

Es ist daher die Aufgabe der vorliegenden Erfindung, wenigstens einen Nachteil des Stands der Technik zumindest teilweise zu überwinden. Insbesondere ist es eine Aufgabe der vorliegenden Erfindung, einen verbesserten Elektrolyten bereitzustellen, der insbesondere einen reduzierten Einsatz an Leitsalz erfordert.

Die Lösung der Aufgabe erfolgt erfindungsgemäß durch einen lösungsmittelbasierten Elektrolyten mit den Merkmalen des Anspruchs 1. Die Lösung der Aufgabe erfolgt ferner durch einen elektrochemischen Energiespeicher mit den Merkmalen des Anspruchs 10 sowie durch eine Verwendung mit den Merkmalen des Anspruchs 11. Bevorzugte Ausgestaltungen der Erfindung sind in den Unteransprüchen, in der Beschreibung und in den Figuren offenbart, wobei weitere in den Unteransprüchen oder in der Beschreibung oder den Figuren beschriebene oder gezeigte Merkmale einzeln oder in einer beliebigen Kombination einen Gegenstand der Erfindung darstellen können, wenn sich aus dem Kontext nicht eindeutig das Gegenteil ergibt.

Beschrieben wird ein lösungsmittelbasierter Elektrolyt für einen elektrochemischen Energiespeicher, aufweisend wenigstens die folgenden Bestandteile:
- wenigstens ein Lösungsmittel; und
- wenigstens ein Leitsalz, wobei
wenigstens ein Lösungsmittel der folgenden Formel (1) entspricht: 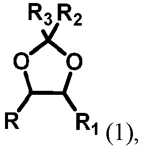 wobei
- R und R₁ gleich oder verschieden sind und ausgewählt sind aus der Gruppe bestehend aus H, CH₃, der Struktur gemäß Formel (3) und der Struktur gemäß Formel (4) oder R und R₁ gemeinsam die Struktur gemäß Formel (2) ausbilden, wobei
- die Formel (2) der folgenden Struktur entspricht: 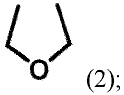
- die Formel (3) der folgenden Struktur entspricht: 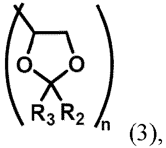 wobei n eine ganze Zahl von 1 bis 10 ist; und
- die Formel (4) der folgenden Struktur entspricht: 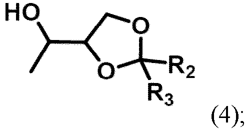 wobei in den Formeln (1), (3) und (4) R₂ und R₃ gleich oder verschieden sind und ausgewählt sind aus der Gruppe bestehend aus H und (CH₂)ₓ-CH₃, wobei x eine ganze Zahl von 0 bis 9 ist.

Es hat sich gezeigt, dass ein derartiger Elektrolyt gegenüber Lösungen aus dem Stand der Technik deutliche Vorteile bietet. Insbesondere bietet die vorliegende Erfindung deutliche Vorteile durch die Verwendung spezifischer Lösungsmittel in Elektrolyten für elektrochemische Energiespeicher.

Die vorliegende Erfindung betrifft einen lösungsmittelbasierten Elektrolyten.

Lösungsmittelbasierte Elektrolyte weisen im Vergleich zu Feststoffelektrolyten eine höhere Leitfähigkeit und einen besseren Kontakt zwischen Elektrolyt und Elektroden auf. Darüber hinaus können durch entsprechende Additive auch bei gängigen organischen Lösungsmitteln, welche gegebenenfalls entflammbar sind, eine Brennbarkeit erschwert und die Gefahr des thermischen Durchgehens reduziert werden. Alternativ ist die Verwendung von lösungsmittelbasierten, wässrigen Elektrolyten möglich, da Elektrolyte mit Wasser als alleinigem oder Co-Lösungsmittel aufgrund der intrinsischen Nichtbrennbarkeit des Wassers ebenfalls Vorteile hinsichtlich der Sicherheit aufweisen und ein thermisches Durchgehen verhindern können.

Insbesondere ist Gegenstand der vorliegenden Erfindung somit eine neue Klasse von Lösungsmitteln zur Verwendung in Elektrolyten von elektrochemischen Energiespeichern, wie etwa von Lithium-Ionen-Batterien, Natrium-Batterien oder Zink-Ionen-Batterien, ohne jedoch die Erfindung hierauf zu beschränken.

Der Elektrolyt umfasst zunächst ein Leitsalz. Das Leitsalz ist nicht grundsätzlich beschränkt, sondern kann beispielsweise ein an sich bekanntes Leitsalz sein, das etwa Lithiumbis(trifluormethylsulfonyl)imid (LiTFSI) oder Lithiumhexafluorophosphat (LiPF₆) umfassen kann. Insbesondere ist das Leitsalz abhängig von der Art des elektrochemischen Energiespeichers, also beispielsweise, ob der elektrochemische Energiespeicher eine Lithium-Ionen-Batterie ist oder auf anderen chemischen Prozessen basiert.

Darüber hinaus weist der Elektrolyt wenigstens ein Lösungsmittel auf. Dabei ist wenigstens ein Lösungsmittel des erfindungsgemäßen Elektrolyten ein zyklisches Acetal, welches der folgenden Formel (1) entspricht: 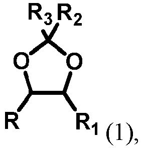 wobei
- R und R1 gleich oder verschieden sind und ausgewählt sind aus der Gruppe bestehend aus H, CH₃, der Struktur gemäß Formel (3) und der Struktur gemäß Formel (4) oder R und R₁ gemeinsam die Struktur gemäß Formel (2) ausbilden, wobei
- die Formel (2) der folgenden Struktur entspricht: 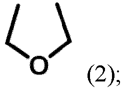
- die Formel (3) der folgenden Struktur entspricht: 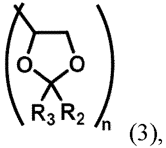 wobei n eine ganze Zahl von 1 bis 10 ist; und
- die Formel (4) der folgenden Struktur entspricht: 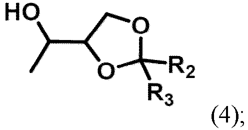 wobei in den Formeln (1), (3) und (4) R₂ und R₃ gleich oder verschieden sind und ausgewählt sind aus der Gruppe bestehend aus H, (CH₂)ₓ-CH₃, wobei x eine ganze Zahl von 0 bis 9, bevorzugt von 0 bis 5, ist, beispielsweise von 1 bis 9, bevorzugt von 1 bis 5. Grundsätzlich kann x somit eine ganze Zahl von größer oder gleich 1, bevorzugt bis 9 sein.

Dabei ist grundsätzlich j egliche Kombination der vorgenannten Reste R, R₁, R₂ und R₃ möglich.

Das vorbeschriebene Lösungsmittel betrifft somit cyclische Acetale, welche insbesondere in Isopropyliden- oder Dioxolan-basierter Modifikation vorliegen können. Derartige Lösungsmittel können aus modifizierten Zuckeralkoholen und damit aus nachwachsenden Rohstoffen und Aldehyden oder Ketonen hergestellt werden und können somit einen natürlichen Ursprung aufweisen. Dies zeigt, dass das Lösungsmittel und damit der Elektrolyt sehr vorteilhaft ist mit Bezug auf die Nachhaltigkeit. Ferner können die definierten Lösungsmittel so problemlos rezyklierbar sein.

Beispiele für entsprechende Edukte umfassen etwa Erythritol, Xylitol oder Sorbitol, welche frei verfügbar und als Edukte nachhaltig und kostengünstig sind.

Weiterhin bieten diese Lösungsmittel den Vorteil, dass Probleme aus dem Stand der Technik, der oftmals insbesondere mit fluorierten Produkten arbeitet, teilweise und in Abhängigkeit von der Wahl des Leitsalzes vollständig verhindert werden können. Dadurch kann die Nachhaltigkeit beziehungsweise die Umweltverträglichkeit weiter verbessert werden.

Dieser Vorteil kann noch dadurch weiter verstärkt werden, dass es die beschriebene Klasse an Lösungsmitteln möglich macht, gegenüber den hochkonzentrierten Lösungen aus dem Stand der Technik, vor allem bei wässrigen Elektrolyten, die Konzentration des Leitsalzes deutlich zu reduzieren. Dadurch kann eine weiter verbesserte Nachhaltigkeit, Ressourcenschonung und ferner eine Reduzierung der Kosten möglich sein.

Darüber hinaus hat sich gezeigt, dass die chemische Struktur der vorstehend definierten Acetale derart ausgeprägt ist, dass die Acetale beziehungsweise das verwendete Lösungsmittel eine hohe chemische Stabilität sowie ein breites elektrochemisches Stabilitätsfenster selbst bis in den Hochvolt-Bereich aufweisen, so dass eine besonders große Anwendungsbreite von mit einem beschriebenen Elektrolyten ausgestatteten elektrochemischen Energiespeichern vorhanden ist.

Weiterhin wurde auf überraschende Weise gefunden, dass die Gesamtleitfähigkeit der Elektrolytformulierungen etwa aufgrund einer moderaten Viskosität sehr vorteilhaft ist und die Elektrolyte ferner eine besonders hohe Teilleitfähigkeit der für die Insertion/Deinsertion relevanten Kationenspezies, wie etwa Lithium-Kationen, aufweisen. Insbesondere kann der Elektrolyt aufweisend das vorstehend definierte Lösungsmittel eine Überführungszahl von größer oder gleich 0,5 aufweisen. Beispielhaft sei hier (3aR, 6aS)-2,2-dimethyltetrahydrofuro[3,4-d][1,3]dioxol (THFD) genannt, welches mit t_{Li}⁺ ~ 0,5 eine deutlich höhere Überführungszahl aufweist als die gängigen Flüssigelektrolyte mit Werten von 0,2-0,4. Entsprechend weisen die beschriebenen Elektrolyte eine besonders hohe Teilleitfähigkeit der für die Insertion/Deinsertion in die Elektroden relevanten Lithiumkationen auf.

Die beschriebenen Lösungsmittel sind ferner auf einfache Weise durch eine einstufige Synthese aus einem Zuckeralkohol und einem Aldehyd oder Keton unter Säurekatalyse erzeugbar. Die Isolierung kann durch eine einfache Aufreinigung möglich sein, so dass die Herstellung problemlos ist. Darüber hinaus kann die Herstellung sehr anpassbar sein schon durch die einfache Auswahl der Edukte. Ferner führt bei gleichem Edukt die Synthese in Abhängigkeit der gewählten Temperatur, beispielsweise Raumtemperatur oder erhöhter Temperatur, etwa 80°C, zu unterschiedlichen Modifikationen, nämlich Isopropyliden- oder Dioxolan-Modifikation des erzeugten cyclischen Acetals, was die Einfachheit der Erzeugung unterschiedlicher Modifikationen zeigt. Schließlich kann durch die problemlose Herstellung, die meist in einem Schritt möglich ist, eine problemlose Skalierbarkeit gegeben sein.

Zur Herstellung des fertigen Elektrolyten kann das beschriebene Lösungsmittel alleine oder unter Verwendung wenigstens eines Co-Lösungsmittels, wie etwa Wasser oder Carbonat-basierten Lösungsmitteln, mit einem Leitsalz und gegebenenfalls mit einem oder mehreren an sich bekannten Additiven kombiniert werden. Dieser Elektrolyt ist dann einsatzbereit und kann in elektrochemischen Energiespeichern eingesetzt beziehungsweise in kommerziellen Batteriezellen galvanostatisch zyklisiert werden.

In einer bevorzugten Ausgestaltung können in der Formel (1) R und R1 einer aus den folgenden Kombinationen entsprechen:
- R ist Hund R₁ ist H;
- R ist CH₃ und R₁ ist CH₃;
- R und R₁ bilden die Struktur gemäß Formel (2) aus;
- R ist Hund R₁ ist die Struktur gemäß Formel (3);
- R ist die Struktur gemäß Formel (3) und R₁ ist die Struktur gemäß Formel (3);
- R ist H und R₁ ist die Struktur gemäß Formel (4).

Diese Ausgestaltungen zeigen besonders vorteilhafte Eigenschaften und sind ferner unter Verwendung gut verfügbarer Edukte erzeugbar.

Gleiches gilt für die Ausgestaltung, wonach in den Formeln (1), (3) und (4) R₂ und R₃ einer aus den folgenden Kombinationen entsprechen:
- R₂ ist Hund R₃ ist H;
- R₂ ist H und R₃ ist (CH₂)ₓ-CH₃; und
- R₂ ist (CH₂)ₓ-CH₃ und R₃ ist (CH₂)ₓ-CH₃, wobei R₂, R₃ und x wie vorstehend definiert sind.

In weiter bevorzugten Ausgestaltungen kann es vorgesehen sein, dass R Wasserstoff ist und R₁ die Struktur gemäß Formel (3) ist oder R und R₁ eine Struktur gemäß Formel (3) sind. In diesen Ausgestaltungen ist für die Struktur gemäß Formel (3) bevorzugt, dass n gleich 1 ist und Formel (3) entsprechend der folgenden Struktur (3a) entspricht:

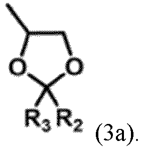

Hierbei können R₂ und R₃ gleich oder verschieden sein und ausgewählt aus der Gruppe bestehend aus H und (CH₂)ₓ-CH₃, wobei x vorzugsweise gleich 0 sein kann. Derartige Lösungsmittel sind aus Edukten wie Erythritol oder Sorbitol nachhaltig und kostengünstig herstellbar. Strukturen gemäß Formel (3) wobei n eine ganze Zahl von 2 bis 10 ist, sind insbesondere synthetisch herstellbar.

In einer besonders bevorzugten Ausgestaltung kann es vorgesehen sein, dass das Lösungsmittel ausgewählt ist aus einer der folgenden Strukturen:
- Struktur (5) 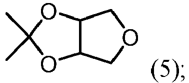
- Struktur (6) 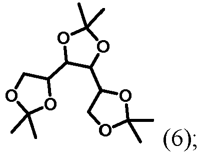 und
- Struktur (7)

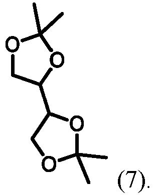

Es hat sich gezeigt, dass die vorgenannten Vorteile insbesondere für diese Substanzen beziehungsweise für diese Lösungsmittel besonders ausgeprägt sein können. Darüber hinaus sind insbesondere diese Produkte problemlos herstellbar, so dass eine Implementierung in bestehende Systeme unproblematisch ist.

In einer weiteren Ausgestaltung kann es bevorzugt sein, dass zusätzlich wenigstens ein weiteres Co-Lösungsmittel vorgesehen ist. Dadurch können die Eigenschaften, wie etwa die ionischen Eigenschaften beziehungsweise die Leitfähigkeitseigenschaften des Elektrolyten weiter maßgeschneidert werden und es können ferner die positiven Eigenschaften unterschiedlicher Lösungsmittel miteinander kombiniert werden. Darüber hinaus kann die brandbasierte Gefahr des Elektrolyten weiter reduziert werden, wenn ein Co-Lösungsmittel mit intrinsischer Nichtbrennbarkeit wie etwa Wasser verwendet wird.

In einer besonders vorteilhaften Ausgestaltung kann es vorgesehen sein, dass Wasser als ein Co-Lösungsmittel vorgesehen ist. Beispielsweise kann das Co-Lösungsmittel nur Wasser aufweisen, so dass als Lösungsmittel nur wenigstens ein vorbeschriebenes cyclisches Acetal und Wasser vorhanden sind. In dieser Ausgestaltung ist der Elektrolyt somit ein wässriger Elektrolyt. Vorteile von wässrigen Elektrolyten können etwa darin gesehen werden, dass diese im Vergleich zu nicht-wässrigen, organischen Elektrolyten eine besonders hohe elektrische Leitfähigkeit aufweisen. Zwar werden für das Konzept der Wasser-in-Salz-Elektrolyten grundsätzlich hohe Salzkonzentrationen beziehungsweise eine hohe Konzentration des Leitsalzes benötigt, um eine ausreichende elektrochemische Stabilität zu erzielen. Dies ist aufgrund des Vorsehens der Mischung aus dem vorbeschriebenen cyclischen Acetal und Wasser als Co-Lösungsmittel jedoch problemlos möglich. Denn es hat sich gezeigt, dass die zyklischen Acetale, welche erfindungsgemäß als Lösungsmittel für den Elektrolyt verwendet werden, sich vorteilhaft dazu eignen, als Co-Lösungsmittel und Diluent von wässrigen Elektrolyten verwendet zu werden und so das elektrochemische Stabilitätsfenster von hinsichtlich des Leitsalzes moderat-konzentrierten Elektrolyten zu erweitern, indem ein lokal hochkonzentrierter Elektrolyt ausgebildet wird. In anderen Worten wird es möglich, dass bei einer Kombination der definierten Acetale mit Wasser als Co-Lösungsmittel das Leitsalz sich weitgehend in der wässrigen Phase befindet. Dadurch kann die Konzentration des Leitsalzes lokal in der wässrigen Phase stark erhöht werden, obgleich grundsätzlich, bezogen auf den gesamten Elektrolyten beziehungsweise bezogen auf das gesamte Lösungsmittel, eine verglichen zu den Lösungen aus dem Stand der Technik sehr niedrige Gesamt-Konzentration des Leitsalzes vorliegt.

Darüber hinaus kann gerade bei der Verwendung von Wasser als Co-Lösungsmittel die Nachhaltigkeit besonders ausgeprägt sein. Denn neben den wie vorstehend beschriebenen diesbezüglichen Vorteilen der zyklischen Acetale ist auch die Verwendung von Wasser hinsichtlich der Nachhaltigkeit besonders vorteilhaft.

In Ausführungsformen der Verwendung von Wasser als Co-Lösungsmittel, kann Wasser in einem Anteil in einem Bereich von 1 Gew.-% bis 25 Gew.-%, vorzugsweise im Bereich von 5 Gew.-% bis 15 Gew.-%, vorzugsweise im Bereich von 5 Gew.-% bis 10 Gew.-%, bezogen auf ein Gesamtgewicht des Lösungsmittels von 100 Gew.-%, vorgesehen sein. In diesen Bereichen kann die Konzentration an Leitsalz im wässrigen Elektrolyten in vorteilhaft niedrigen Bereichen liegen.

Beispielsweise aber nicht grundsätzlich beschränkt auf die Verwendung von Wasser als Co-Lösungsmittel kann das Leitsalz in dem Elektrolyten in einer Menge vorliegen von ≤ 5 mol/kg, bezogen auf das vorhandene Lösungsmittel, also sämtlicher vorhandener Lösungsmittel. Beispielsweise kann das Leitsalz in einem Bereich vorliegen von ≤ 3 mol/kg, etwa in einem Bereich von ≥ 0,5 mol/kg bis ≤ 5 mol/kg, beispielsweise in einem Bereich von ≥ 0,5 mol/kg bis ≤ 5 mol/kg. Dadurch kann die Nachhaltigkeit des erfindungsgemäßen Elektrolyten weiter verbessert werden. Darüber hinaus kann ein derartiger Elektrolyt besonders ressourcenschonend und kostengünstig herstellbar sein. Der Vorteil dieser Ausgestaltung zeigt sich besonders für wässrige Elektrolyte dann, wenn beachtet wird, dass Lösungen aus dem Stand der Technik meist mit Konzentrationen des Leitsalzes von ungefähr 21 mol/ kg an Leitsalz arbeiten. Entsprechend wird insbesondere in dieser Ausgestaltung deutlich, dass die Konzentration an Leitsalz grundsätzlich gegenüber den Lösungen aus dem Stand der Technik deutlich reduziert werden kann.

Es kann weiterhin von Vorteil sein, dass das Leitsalz Lithiumbis(trifluormethylsulfonyl)imid (LiTFSI) oder Lithiumhexafluorophosphat (LiPF₆) umfasst. Insbesondere diese Leitsalze sind in elektrochemischen Energiespeichern bewährt und weisen eine gute Performance auf. Darüber hinaus hat sich gezeigt, dass die definierten Lösungsmittel gerade für diese Leitsalze ein vorteilhaftes Insertions-/Desinsertionsverhalten aufweisen.

Hinsichtlich weiterer Vorteile und technischer Merkmale des Elektrolyten wird verwiesen auf die Beschreibung des elektrochemischen Energiespeichers, der Verwendung, der Figuren und der Beschreibung der Figuren.

Weiter beschrieben ist ein elektrochemischer Energiespeicher, aufweisend eine Anode, eine Kathode und einen zwischen der Anode und der Kathode angeordneten Elektrolyten, wobei der Elektrolyt ein lösungsmittelbasierter Elektrolyt wie vorstehend beschrieben ist.

Der Energiespeicher umfasst somit in an sich bekannter Weise eine Anode und eine Kathode, welche grundsätzlich wie für die entsprechende Batterie bekannt ausgestaltet sein können. Zwischen den Elektroden und diese verbindend ist ferner ein Elektrolyt vorgesehen. Dieser ist ausgebildet wie vorstehend beschrieben.

Es ergeben sich somit die zuvor beschriebenen Vorteile, nämlich insbesondere besonders vorteilhafte elektrische und ionische Eigenschaften, ein möglicher deutlich reduzierter Gehalt an Leitsalz und ferner eine besonders umweltfreundliche und nachhaltige Ausgestaltung.

Hinsichtlich weiterer Vorteile und technischer Merkmale des elektrochemischen Energiespeichers wird verwiesen auf die Beschreibung des Elektrolyten, der Verwendung, der Figuren und der Beschreibung der Figuren.

Gemäß der vorliegenden Beschreibung weiterhin Gegenstand der vorliegenden Erfindung die Verwendung eines Lösungsmittels in einem lösungsmittelbasierten Elektrolyten für einen elektrochemischen Energiespeicher, wobei das Lösungsmittel der folgenden Formel (1) entspricht: 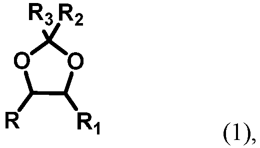 wobei
- R und R₁ gleich oder verschieden sein können und ausgewählt sind aus der Gruppe bestehend aus H, CH₃, der Struktur gemäß Formel (3) und der Struktur gemäß Formel (4) oder R und R₁ gemeinsam die Struktur gemäß Formel (2) ausbilden, wobei
- die Formel (2) der folgenden Struktur entspricht: 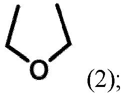
- die Formel (3) der folgenden Struktur entspricht: 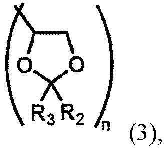 wobei n eine ganze Zahl von 1 bis 10 ist;
- die Formel (4) der folgenden Struktur entspricht: 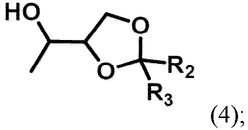 wobei in den Formeln (1), (3) und (4) R₂ und R₃ gleich oder verschieden sind und ausgewählt sind aus der Gruppe bestehend aus H, (CH₂)ₓ-CH₃, wobei x eine ganze Zahl von 0 bis 9 ist.

Es ergeben sich somit die zuvor beschriebenen Vorteile, nämlich insbesondere besonders vorteilhafte elektrische und ionische Eigenschaften, ein möglicher deutlich reduzierter Gehalt an Leitsalz und ferner eine besonders umweltfreundliche und nachhaltige Ausgestaltung.

Hinsichtlich weiterer Vorteile und technischer Merkmale der Verwendung wird verwiesen auf die Beschreibung des Elektrolyten, des elektrochemischen Energiespeichers, der Beispiele, Figuren und der Beschreibung der Figuren.

Nachfolgend wird die Erfindung unter Bezugnahme auf die anliegenden Zeichnungen exemplarisch erläutert, wobei die nachfolgend dargestellten Merkmale sowohl jeweils einzeln als auch in Kombination einen Aspekt der Erfindung darstellen können, und wobei die Erfindung nicht auf die folgende Zeichnung, die folgende Beschreibung und das folgende Ausführungsbeispiel beschränkt ist:
Fig. 1 zeigt schematisch eine beispielhafte Synthese von zyklischen Acetalen;
Fig. 2 zeigt das Diagramm einer Cyclovoltammetrie;
Fig. 3 zeigt Abscheidungs- und Auflösungstestmessungen eines Elektrolyten;
Fig. 4 zeigt weitere Abscheidungs- und Auflösungstestmessungen des Elektrolyten aus Fig. 3;
Fig. 5 zeigt das Diagramm einer Cyclovoltammetrie;
Fig. 6 zeigt das Diagramm von DSC-Messungen;
Fig. 7 zeigt verschiedene IR-Spektrogramme;
Fig. 8 zeigt die Ergebnisse einer Leitfähigkeitsmessung für zwei Elektrolyte; und
Fig. 9 zeigt die Ergebnisse einer galvanostatischen Zyklisierung eines der Elektrolyte aus Fig. 8.

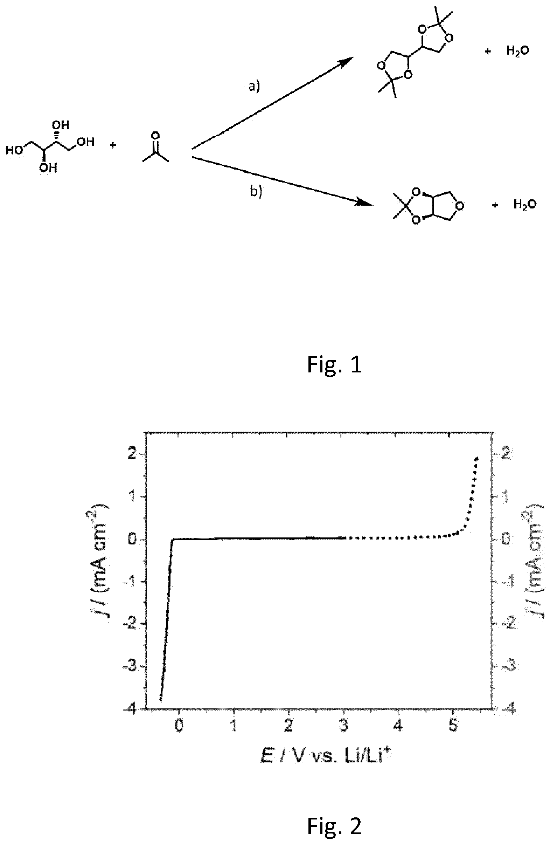

In der Figur 1 ist schematisch die Synthese von zyklischen Acetalen gemäß einer Ausgestaltung der vorliegenden Erfindung gezeigt, welche als Lösungsmittel in einem Elektrolyten für einen elektrochemischen Energiespeicher verwendbar sind. Genauer zeigt die Figur 1 die Herstellung von zyklischen Acetalen ausgehend von Erythritol und Aceton. Je nach Herstellungsbedingungen können unterschiedliche Modifikationen erzeugt werden. Bei Raumtemperatur können bei einer Reaktionsdauer von 18 Stunden säurekatalysiert, wie etwa mit Schwefelsäure, gemäß Route a) 2, 2, 2', 2'-tetramethyl-4,4'-bi(1,3-dioxolan) und Wasser erzeugt werden und bei erhöhter Temperatur, beispielsweise 120 °C, und einer Reaktionsdauer von 24 Stunden und unter Verwendung von p-Toluolsulfonsäure, beispielsweise können gemäß Route b) (3aR, 6aS)-2,2-dimethyltetrahydrofuro[3,4-d][1,3]dioxol (THFD) und Wasser erzeugt werden.

Figur 2 bezieht sich auf ein Beispiel für einen Elektrolyten mit THFD, der gemäß Route b) aus Figur 1 herstellbar ist, als alleiniges Lösungsmittel. Im Detail wurde eine Mischung von 0.7 mol kg⁻¹ LiTFSI als Leitsalz in THFD verwendet. Dabei ist in der Figur 2 ein Diagramm darstellend das Ergebnis einer Cyclovoltammetrie gezeigt, wobei die X-Achse das Potential gegen Li⁺|Li und die Y-Achse die Oberflächenstromdichte zeigt. Das elektrochemische Stabilitätsfenster wurde gegen Inertmaterial bestimmt mit einer Vorschubgeschwindigkeit von 1 mV s⁻¹ bei 20 °C. Im reduktiven Bereich wurde auf Kupfer als Arbeitselektrode gemessen, was durch die durchgehende Kurve gezeigt wird. Der Elektrolyt zeigt hier eine Stabilität bis zur Lithiumabscheidung. Im oxidativen Bereich, der auf Platin gemessen wurde und durch die punktierte Kurve dargestellt ist, zeigt sich eine Stabilität bis 5,2 V vs. Li⁺|Li. Entsprechend beträgt das elektrochemische Stabilitätsfenster 5,2 V, was eine Anwendung in Kombination mit Hochvolt-Elektrodenmaterialien ermöglicht.

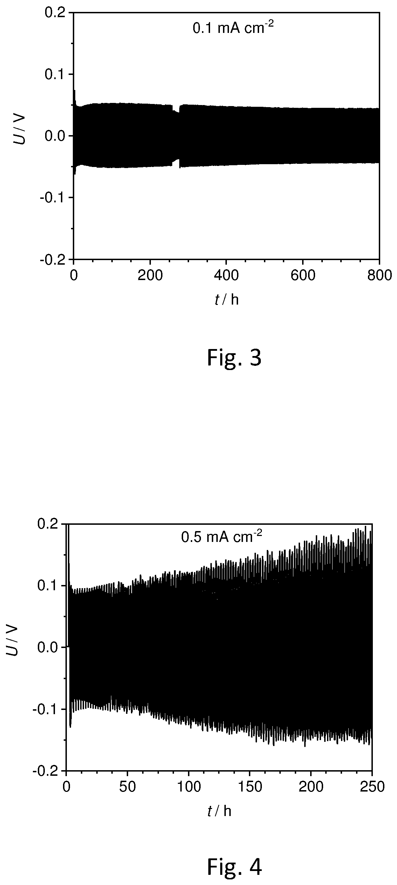

In den Figuren 3 und 4 sind ferner Abscheidungs- und Auflösungstestmessungen des Elektrolyten in einer symmetrischen Lithiumzelle bei 0,1 mA cm⁻² (Figur 3) bzw. 0,5 mA cm⁻² (Figur 4) gezeigt, die eine stabile Zyklisierung mit geringer Überspannung über 400 bzw. 125 Zyklen zeigen. Dabei wurde je eine Stunde geladen beziehungsweise entladen. Dies zeigt die Langzeitstabilität des Elektrolyten, der wiederum eine Mischung von 0.7 mol kg⁻¹ LiTFSI als Leitsalz in THFD umfasst hat.

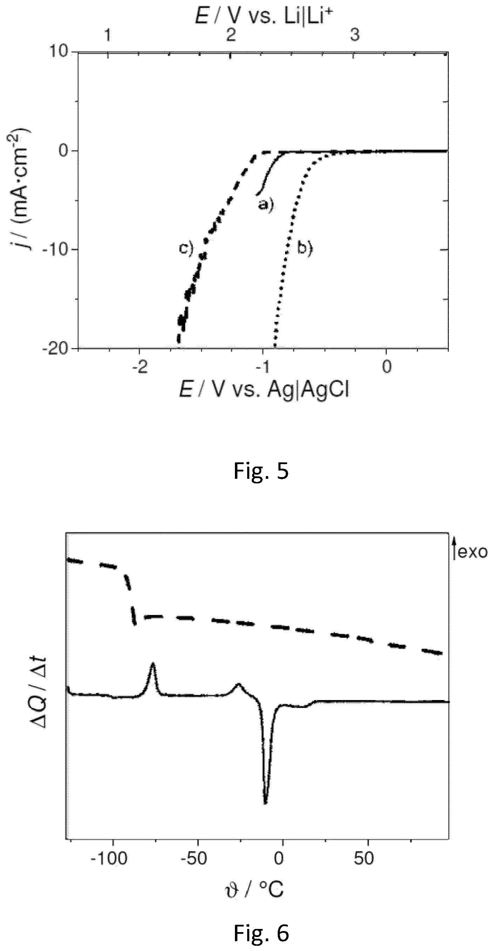

In der Figur 5 ist die Anwendung von Acetalen als Co-Lösungsmittel bzw. Diluenten für wässrige Elektrolyte gezeigt. Dabei zeigt die Kurve a) 21 molales LiTFSI in Wasser, zeigt die Kurve b) 1 molales LiTFSI in einer Mischung aus Wasser mit 36 Gew.-% Erythritol und zeigt die Kurve c) 2,1 molares LiTFSI in einer Mischung aus Wasser mit THFD in einem Verhältnis in Gewichtsanteilen Wasser:THFD von 1:9. Ferner zeigt die X-Achse das Potential, unten gegen Ag|AgCl und oben gegen Li⁺|Li und zeigt die Y-Achse die Oberflächenstromdichte. In dem Diagramm der Cyclovoltammetrie gemäß Figur 5 wurde das elektrochemische Stabilitätsfenster gegen Platin als Inertmaterial bestimmt mit einer Vorschubgeschwindigkeit von 1 mV s⁻¹ bei 20 °C. In Figur 5 ist nur der reduktive Bereich gezeigt, wo erkennbar ist, dass der Elektrolyt mit Acetal als Co-Lösungsmittel im Vergleich zum moderat-konzentrierten 1 m LiTFSI in H₂O und dem Referenzelektrolyten 21 m LiTFSI in H₂O die Wasserstofffreisetzung durch Reduktion der Wassermoleküle an der Elektrodenoberfläche erst bei niedrigeren Potentialen auftritt. Insgesamt ergibt sich ein elektrochemisches Stabilitätsfenster von > 3V für diesen und den Referenzelektrolyten. Dies zeigt, dass dadurch trotz Reduzierung der Menge an Leitsalz im Elektrolyten mit THFD ein breites elektrochemisches Stabilitätsfenster, welches für wässrige Elektrolyte im Gegensatz zu organischen Elektrolyten bereits relativ groß ist, erzielt werden kann.

In der Figur 6 ist ein Diagramm einer Differenzkalorimetrie (DSC)-Messung bei einer Heizrate von 10 K min⁻¹ gezeigt. Dabei zeigt die Kurve a) 21 molales LiTFSI in Wasser und zeigt die Kurve b) 2,1 molales LiTFSI in einer Mischung aus Wasser mit THFD in einem Verhältnis in Gewichtsanteilen Wasser:THFD von 1:9. Es ist zu erkennen, dass, während der Referenzelektrolyt (Kurve a)) bei -10 °C schmilzt/kristallisiert, der erfindungsgemäße Elektrolyt (Kurve b) nur einen Glasübergangspunkt bei -90 °C hat. Das bedeutet, dass der erfindungsgemäße Elektrolyt lediglich ein teilkristallines Gitter ausbildet und dies bei einer deutlich niedrigeren Temperatur, was wiederum für eine Vergrößerung des Anwendungsbereichs für die wässrigen Elektrolyte spricht.

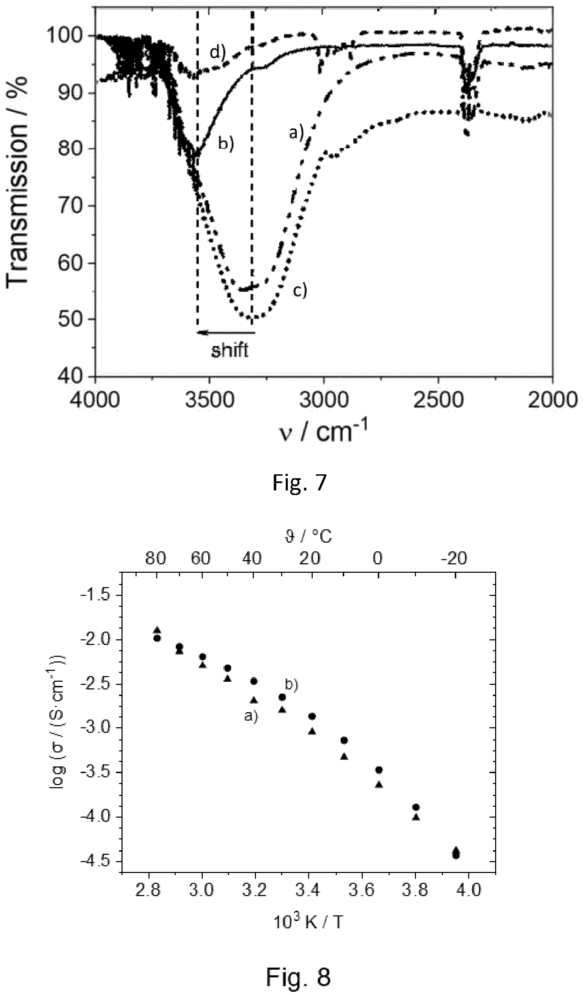

Die Figur 7 zeigt ein IR-Spektrogramm (ATR-FTIR). Dabei zeigt die Kurve a) 1 molales LiTFSI in Wasser, zeigt die Kurve b) 21 molales LiTFSI in Wasser, zeigt die Kurve c) 1 molales LiTFSI in einer Mischung aus Wasser mit 36 Gew.-% Erythritol und zeigt die Kurve d) 2,1 molales LiTFSI in einer Mischung aus Wasser mit THFD in einem Verhältnis in Gewichtsanteilen Wasser:THFD von 1:9. Es ist zu erkennen, dass im Vergleich zum moderat-konzentrierten Elektrolyten (1 m LiTFSI in H₂O) der Elektrolyt mit Acetal als Co-Lösungsmittel (2,1 m LiTFSI in H₂O-THFD (1:9)) analog zum literaturbekannten 21 m LiTFSI in H₂O Elektrolyten eine Verschiebung zu höheren Wellenzahlen zeigt (Shift). Dies deutet auf einen zunehmenden Anteil an Koordination von Wassermolekülen durch Lithiumkationen anstelle von weiteren Wassermolekülen hin, wodurch die Verbreiterung des elektrochemischen Stabilitätsfenster beobachtet werden kann. Diesbezüglich sei angemerkt, dass das LiTFSI aufgrund der Polaritätsunterschiede der beiden Lösungsmittel vermehrt in der wässrigen Phase angereichert werden wird, wobei sich ein lokal hochkonzentrierter Elektrolyt ausbildet. Wichtig ist, dass dieses Konzept des lokal hochkonzentrierten Elektrolyten die drastische Reduzierung der Leitsalzmenge (Faktor 10; 21 molal auf 2,1 molal) ermöglicht, so dass das Leitsalz in einer Konzentration von 0.5 molal bis zu 5 molal vorliegen kann. Damit wird die Verwendung von Wasser in einem breiteren elektrochemischen Stabilitätsfenster ermöglicht.

Figur 8 zeigt die Leitfähigkeit von Elektrolyten enthaltend Acetal und Wasser als Co-Lösungsmittel. Die Leitfähigkeit einer jeweils 2,1 molaren Lösung von LiTFSI in einer Mischung aus Wasser mit THFD in einem Verhältnis in Gewichtsanteilen THFD:Wasser von 9:1 oder einer Mischung aus Wasser mit 2, 2, 2', 2'-tetramethyl-4,4'-bi(1,3-dioxolan) (IPE, Isopropylidenerythritol) gemäß Struktur (7) in einem Verhältnis in Gewichtsanteilen IPE:Wasser: von 9:1 wurde in einem Temperaturbereich von 80°C bis -20°C bestimmt. 2, 2, 2', 2'-tetramethyl-4,4'-bi(1,3-dioxolan) wurde ausgehend von Erythritol und Aceton in einer mit Schwefelsäure katalysierten Reaktion bei Raumtemperatur und einer Reaktionsdauer von 18 Stunden gemäß Route a) wie in Figur 1 gezeigt hergestellt.

Die Leitfähigkeit der Elektrolyte wurde unter Verwendung einer Leitfähigkeitsmesszelle mit ionenblockierenden Multi-Mode-Elektroden aus Edelstahl bestimmt, wobei die Leitfähigkeitsmesszelle zunächst auf -20°C gekühlt und dann in Temperaturintervallen von 10°C auf 80°C erwärmt wurde. Die Impedanzmessungen zur Bestimmung des Widerstands wurden in einem Frequenzbereich von 10⁶ Hz bis 1 Hz bei einer Wechselspannung von 10 mV durchgeführt. Die Zellkonstante der verwendeten Zellen wurde zuvor durch Kalibration mit standarisierter 1 M wässriger Kaliumchlorid-Lösung bestimmt und betrug 4,7 1/cm. Die Figur 8 zeigt den logarithmischen Verlauf der Leitfähigkeit der Elektrolyte a) 2,1 m LiTFSI in THFD:Wasser in Gewichtsanteilen 9:1 sowie b) 2,1 m LiTFSI in IPE:Wasser in Gewichtsanteilen 9:1 für den Temperaturbereich von 80°C bis -20°C. Beide Elektrolyte enthaltend von Erythrit ausgehend synthetisierte Acetale mit Wasser als Co-Lösungsmittel zeigten eine vergleichbare Leitfähigkeit wie man der Figur 8 entnehmen kann.

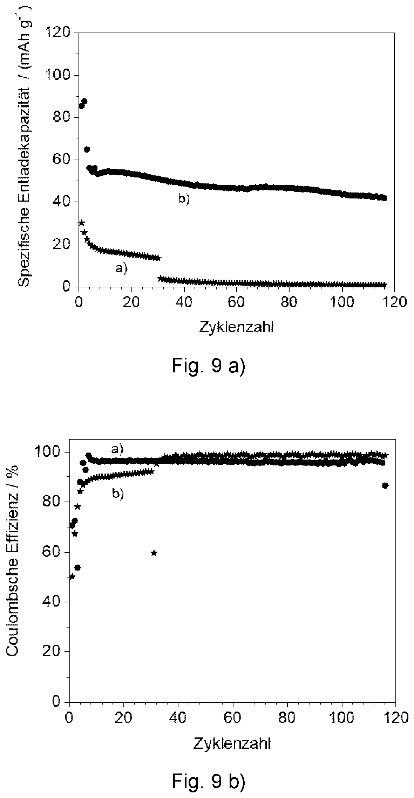

Figur 9 illustriert das Zyklisierverhalten von Elektrolyten enthaltend 2,1 m LiTFSI in Gemischen von Wasser mit (3aR, 6aS)-2,2-dimethyltetrahydrofuro[3,4-d][1,3]dioxol. Hierfür wurden galvanostatische Zyklisierungen mit einem Elektrolyten enthaltend 2,1 m LiTFSI in THFD:Wasser in Gewichtsanteilen 9:1 durchgeführt. Als Referenzelektrolyt wurde 21 molales LiTFSI in Wasser zyklisiert. Die Versuche wurden in einer Elektrodenanordnung mit einer Lithiummanganoxid (LMO)-Elektrode gegen eine Aktivkohle-Elektrode durchgeführt. Die Flächenkapazität der Elektroden betrug 2 mAh/cm². Die galvanostatische Zyklisierung wurde bei einer Temperatur 20 °C und mit Lade- und Entladeraten von C/10 in einem Spannungsbereich von 1,0 V bis 2,5 V durchgeführt.

In der Figur 9a) ist die spezifische Entladekapazität und in Figur 9b) die Coulombsche Effizienz der Elektrolyten a) 21 molales LiTFSI in Wasser und b) 2,1 m LiTFSI in THFD:Wasser in Gewichtsanteilen 9:1 gegen die Zyklenzahl abgebildet. Es ist erkennbar, dass der Elektrolyt enthaltend Acetal und Wasser als Co-Lösungsmittel trotz der deutlich reduzierten Leitsalzmenge von 2,1 molal LiTFSI gegenüber 21 molal LiTFSI eine deutlich höhere spezifische Entladekapazität und Coulombsche Effizienz der galvanostatischen Zyklisierung zeigte. Insbesondere zeigte der Elektrolyt enthaltend 2,1 m LiTFSI in THFD:Wasser eine sehr stabile Zyklisierung, da kaum Kapazitätsverlust zu beobachten ist.

## Patentansprüche

1. Lösungsmittelbasierter Elektrolyt für einen elektrochemischen Energiespeicher, aufweisend wenigstens die folgenden Bestandteile:
- wenigstens ein Lösungsmittel; und
- wenigstens ein Leitsalz, wobei
wenigstens ein Lösungsmittel der folgenden Formel (1) entspricht: 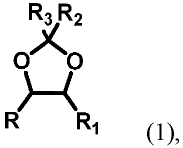
wobei
- R und R₁ gleich oder verschieden sind und ausgewählt sind aus der Gruppe bestehend aus H, CH₃, der Struktur gemäß Formel (3) und der Struktur gemäß Formel (4) oder R und R₁ gemeinsam die Struktur gemäß Formel (2) ausbilden, wobei
- die Formel (2) der folgenden Struktur entspricht: 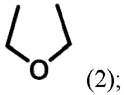
- die Formel (3) der folgenden Struktur entspricht: 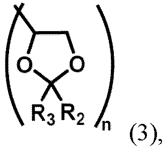 wobei n eine ganze Zahl von 1 bis 10 ist;
- die Formel (4) der folgenden Struktur entspricht: 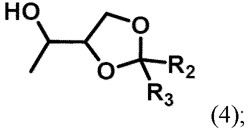
wobei in den Formeln (1), (3), und (4) R₂ und R₃ gleich oder verschieden sind und ausgewählt sind aus der Gruppe bestehend aus H und (CH₂)ₓ-CH₃, wobei x eine ganze Zahl von 0 bis 9 ist.

2. Lösungsmittelbasierter Elektrolyt nach Anspruch 1, **dadurch gekennzeichnet, dass** in der Formel (1) R und R₁ einer aus den folgenden Kombinationen entsprechen:
- R ist Hund R₁ ist H;
- R ist CH₃ und R₁ ist CH₃;
- R und R₁ bilden die Struktur gemäß Formel (2) aus;
- R ist Hund R₁ ist die Struktur gemäß Formel (3);
- R ist die Struktur gemäß Formel (3) und R₁ ist die Struktur gemäß Formel (3);
- R ist H und R₁ ist die Struktur gemäß Formel (4).

3. Lösungsmittelbasierter Elektrolyt nach einem der Ansprüche 1 oder 2, **dadurch gekennzeichnet, dass** in den Formeln (1), (3) und (4) R₂ und R₃ einer aus den folgenden Kombinationen entsprechen:
- R₂ ist Hund R₃ ist H;
- R₂ ist H und R₃ ist (CH₂)ₓ-CH₃; und
- R₂ ist (CH₂)ₓ-CH₃ und R₃ ist (CH₂)ₓ-CH₃.

4. Lösungsmittelbasierter Elektrolyt nach einem der Ansprüche 1 bis 3, **dadurch gekennzeichnet, dass** das Lösungsmittel ausgewählt ist aus einer der folgenden Strukturen:
- Struktur (5) 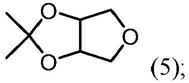
- Struktur (6) 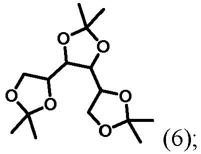 und
- Struktur (7)

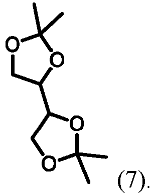

5. Lösungsmittelbasierter Elektrolyt nach einem der Ansprüche 1 bis 4, **dadurch gekennzeichnet, dass** zusätzlich wenigstens ein weiteres Co-Lösungsmittel vorgesehen ist.

6. Lösungsmittelbasierter Elektrolyt nach Anspruch 5, **dadurch gekennzeichnet, dass** Wasser als Co-Lösungsmittel vorgesehen ist.

7. Lösungsmittelbasierter Elektrolyt nach Anspruch 6, **dadurch gekennzeichnet, dass** Wasser als Co-Lösungsmittel in einem Anteil im Bereich von 1 Gew.-% bis 25 Gew.-%, vorzugsweise im Bereich von 5 Gew.-% bis 15 Gew.-%, vorzugsweise im Bereich von 5 Gew.-% bis 10 Gew.-%, bezogen auf ein Gesamtgewicht des Lösungsmittels von 100 Gew.-%, vorgesehen ist.

8. Lösungsmittelbasierter Elektrolyt nach einem der Ansprüche 1 bis 7, **dadurch gekennzeichnet, dass** das Leitsalz in dem Elektrolyten in einer Menge vorliegt von ≤ 5 mol/kg.

9. Lösungsmittelbasierter Elektrolyt nach einem der Ansprüche 1 bis 8, **dadurch gekennzeichnet, dass** das Leitsalz Lithiumbis(trifluormethylsulfonyl)imid umfasst.

10. Elektrochemischer Energiespeicher, aufweisend eine Anode, eine Kathode und einen zwischen der Anode und der Kathode angeordneten Elektrolyten, **dadurch gekennzeichnet, dass** der Elektrolyt ein lösungsmittelbasierter Elektrolyt nach einem der Ansprüche 1 bis 9 ist.

11. Verwendung eines Lösungsmittels in einem lösungsmittelbasierten Elektrolyten für einen elektrochemischen Energiespeicher, **dadurch gekennzeichnet, dass** das Lösungsmittel der folgenden Formel (1) entspricht: 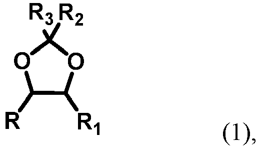 wobei
- R und R1 gleich oder verschieden sein können und ausgewählt sind aus der Gruppe bestehend aus H, CH₃, der Struktur gemäß Formel (3) und der Struktur gemäß Formel (4) oder R und R₁ gemeinsam die Struktur gemäß Formel (2) ausbilden, wobei
- die Formel (2) der folgenden Struktur entspricht: 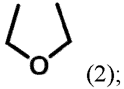
- die Formel (3) der folgenden Struktur entspricht: 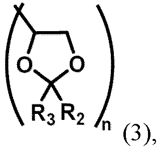 wobei n eine ganze Zahl von 1 bis 10 ist;
- die Formel (4) der folgenden Struktur entspricht: 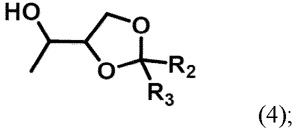
wobei in den Formeln (1), (3) und (4) R₂ und R₃ gleich oder verschieden sind und ausgewählt sind aus der Gruppe bestehend aus H, (CH₂)ₓ-CH₃, wobei x eine ganze Zahl von 0 bis 9 ist.
